(19) Europäisches Patentamt
European Patent Office
Office européen des brevets

(11) **EP 1 886 130 B1**

(12) **EUROPÄISCHE PATENTSCHRIFT**

(45) Veröffentlichungstag und Bekanntmachung des Hinweises auf die Patenterteilung:
**18.09.2013 Patentblatt 2013/38**

(21) Anmeldenummer: **06724358.4**

(22) Anmeldetag: **15.04.2006**

(51) Int Cl.:
***G01N 29/00*** *(2006.01)*

(86) Internationale Anmeldenummer:
**PCT/EP2006/003484**

(87) Internationale Veröffentlichungsnummer:
**WO 2006/128520 (07.12.2006 Gazette 2006/49)**

(54) **VERFAHREN UND VORRICHTUNG ZUM ÜBERWACHEN EINER STRÖMENDEN FLÜSSIGKEIT AUF DAS VORHANDENSEIN VON LUFT**

METHOD AND DEVICE FOR MONITORING A FLOWING LIQUID FOR THE PRESENCE OF AIR

PROCEDE ET DISPOSITIF POUR SURVEILLER UN LIQUIDE EN CIRCULATION, POUR DETECTER LA PRESENCE D'AIR

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **03.06.2005 DE 102005025515**

(43) Veröffentlichungstag der Anmeldung:
**13.02.2008 Patentblatt 2008/07**

(73) Patentinhaber: **Fresenius Medical Care Deutschland GmbH**
**61352 Bad Homburg v.d.H. (DE)**

(72) Erfinder:
• **BALSCHAT, Klaus**
**97525 Schwebheim (DE)**
• **FRITSCHE, Tobias**
**06114 Halle (DE)**
• **GAGEL, Alfred**
**96123 Litzendorf (DE)**
• **KIRCHNER, Steffen**
**97422 Schweinfurt (DE)**
• **MÜNCH, Hans-Joachim**
**06130 Halle (DE)**
• **NICHOLAS, Olaf**
**97318 Kiltzingen (DE)**
• **SCHNEIDER, Jochen**
**97537 Wipfeld (DE)**
• **SPICKERMANN, Rainer**
**97535 Wasserlosen-Burghausen (DE)**
• **ZUR HOST-MEYER, Santer**
**06120 Halle (DE)**

(74) Vertreter: **Oppermann, Frank et al**
**OANDO Oppermann & Oppermann LLP**
**John-F.-Kennedy-Straße 4**
**65189 Wiesbaden (DE)**

(56) Entgegenhaltungen:
EP-A- 0 643 301        DE-A1- 4 013 402
US-A- 4 651 555        US-A- 5 824 881
US-A1- 2004 154 374

**Beschreibung**

Die Erfindung bezieht sich auf ein Verfahren zum Überwachen einer strömenden Flüssigkeit auf das Vorhandensein von Luft, insbesondere des in einem extrakorporalen Blutkreislauf einer extrakorporalen Blutbehandlungsvorrichtung strömenden Blutes, sowie ein Verfahren zur extrakorporalen Blutbehandlung mit einem extrakorporalen Blutkreislauf, wobei das Vorhandensein von Luft in dem extrakorporalen Blutkreislauf überwacht wird. Darüber hinaus betrifft die Erfindung eine Vorrichtung zum Überwachen einer strömenden Flüssigkeit, insbesondere des in einem extrakorporalen Kreislauf strömenden Blutes, auf das Vorhandensein von Luft, sowie eine Vorrichtung zur extrakorporalen Blutbehandlung mit einer Vorrichtung zur Überwachung des in dem extrakorporalen Kreislauf der Blutbehandlungsvorrichtung strömenden Blutes auf das Vorhandensein von Luft.

Es sind verschiedene Verfahren zur extrakorporalen Blutbehandlung bekannt, bei denen das Blut des Patienten in einem extrakorporalen Blutkreislauf durch eine Blutbehandlungseinheit strömt. Eine der wesentlichen Komplikationen bei der extrakorporalen Blutbehandlung, beispielsweise Hämodialyse oder Hämofiltration, stellt die Möglichkeit des Eindringens von Luft in den extrakorporalen Blutkreislauf dar. Das gleiche Risiko besteht nicht nur bei extrakorporalen Blutbehandlungen, sondern auch bei Infusionen mit Infusionslösungen.

Zum Abscheiden mitgeführter Luftblasen aus Blut oder Infusionslösungen dienen die bekannten Tropfkammern, die im venösen Zweig des extrakorporalen Blutkreislaufs bzw. in der Infusionsleitung angeordnet werden. Die bekannten Tropfkammern fangen die Luftblasen mit hoher Sicherheit ab. Dennoch besteht grundsätzlich die Gefahr, dass Luftblasen dem Patienten intravenös infundiert werden. Für Blutbehandlungsvorrichtungen schreibt daher die DIN/EN 60601-2-16 zur weiteren Erhöhung der Sicherheit Luftdetektoren vor, an deren Funktionssicherheit sehr hohe Anforderungen gestellt werden. Die bekannten Luftdetektoren beruhen auf der unterschiedlichen Absorption von Ultraschall in flüssigen und gasförmigen Medien sowie der Streuung von Ultraschall an Grenzflächen. Neben den Ultraschallsensoren sind Luftdetektoren bekannt, die auf den unterschiedlichen Dielektrizitätskonstanten und den unterschiedlichen Leitfähigkeiten von flüssigen und gasförmigen Medien basieren. Zur Detektion von Luft werden Signalimpulse in die strömende Flüssigkeit eingekoppelt, während die aus der strömenden Flüssigkeit austretenden Signalimpulse empfangen werden. Dabei wird auf das Vorhandensein von Luft dann geschlossen, wenn das Empfangssignal einen oder mehrere feste Referenzpegel unterschreitet.

Um das Messergebnis nicht zu verfälschen, müssen Änderungen der Umgebungsbedingungen, die Einfluss auf das Empfangssignal haben, kompensiert werden. Hierfür finden verschiedene Kompensationsverfahren Verwendung. Eine Vorrichtung zur Erkennung von Luftblasen in strömenden Flüssigkeiten, die auf einer Ultraschallmessung mit einer Kompensation der Umgebungseinflüsse beruht, ist beispielsweise aus der EP 1 182 452 A2 bekannt.

Die bekannten Verfahren zum Überwachen von strömenden Flüssigkeiten auf das Vorhandensein von Luft haben sich in der Praxis zur Detektion von relativ großen Luftblasen bewährt. Denn die relativ großen Luftblasen, die als Einzelblasen ein Volumen von ca. 1 $\mu$l und als Bolus von ca. 50 $\mu$l überschreiten, verursachen relativ kurze und große Signalveränderungen. Daher können die Einflüsse der sich dazu relativ langsam ändernden Umgebungseinflüsse leicht erkannt werden. So erfolgt die Ausregelung der Umgebungseinflüsse in einer wesentlich größeren Zeitdauer, als die Dauer der auf die Luftblasen zurückzuführenden Signaländerungen.

Neben den relativ großen Einzelblasen können beispielsweise bei Dialysebehandlungen auch sehr kleine Luftbläschen, sogenannte Mikroblasen, auftreten. Sie entstehen typischerweise, wenn Luft aufgrund von Undichtigkeiten in den arteriellen Zweig des extrakorporalen Blutkreislaufs eindringen kann. Die in der Regel am Anfang noch relativ großen Einzelblasen werden zunächst durch die Blutpumpe zerkleinert. Beim anschließenden Durchgang durch die Kapillaren des Dialysators, die im Allgemeinen einen Innendurchmesser von ca. 0,2 mm haben, werden die Luftbläschen noch weiter zerkleinert, Die größeren Luftbläschen können im venösen Blasenfänger aufgrund ihrer Auftriebskraft abgeschieden werden, während die Mikroblasen, die im Allgemeinen einen Durchmesser von ca. 0,2 mm und ein Volumen von ca. 4nl haben, mit dem Blutstrom aus dem Blasenfänger transportiert werden und zum Patienten gelangen können. Im Blutstrom des Patienten befinden sich dann Mikroblasen mit einer kontinuierlichen Durehmesserverteilung mit einem maximalen Durchmesser von ca. 0,3 mm.

Vor einer Dialysebehandlung wird der extrakorporale Blutkreislauf in der Regel mit einer isotonischen Kochsalzlösung gespült. Dabei besteht die Gefahr, dass nicht freigespülte Luftblasen sich während der Behandlung lösen und dann in einer "Wolke" aus Mikroblasen in den Patienten unerkannt infundiert werden können.

In der Literatur (Droste DW, Kühne K, Schaefer RM, Ringelstein EB. Detection of microemboli in the subclavian vein of patients undergoing haemodialysis an haemodiafiltration using pulsed Doppler ultrasound. Nephrol Dial Transplant 2002; 17:462-466) gibt es Hinweise, dass in Dialysebehandlungen typischerweise Mikroembolien auftreten, deren Ursache in dem Vorhandensein von Mikroblasen vermutet wird. Der menschliche Körper toleriert zwar relativ große Luftmengen, wenn sie intravenös verabreicht werden, da die Luft in der Lunge abgeatmet oder im Blut gelöst werden kann, In der Praxis wird eine kontinuierliche Luftinfusionsrate bis 1,5 ml/min bei einem Körpergewicht von 50 kg und einem maximalen Blutfluss von 600 ml/min akzeptiert. Sollten diese Grenzwerte jedoch überschritten werden, kann es zu ernsthaften Komplikationen während der Blutbehandlung kommen.

Die US 5,824,881 beschreibt ein Verfahren zum Überwachen eines strömenden Mediums auf das Vorhandensein von Luft, bei dem eine Folge von Signalimpulsen in das strömende Medium eingekoppelt und eine Folge von Signalimpulsen aus dem strömenden Medium ausgekoppelt werden. Aus dem ausgekoppelten Signal wird eine Detektion-Signatur ermittelt, die aus Gründen der Redundanz mit zwei Referenzsignaturen verglichen wird. Die Referenz-Signatur soll ein Zustand beschreiben, bei dem Luftblasen in der Leitung nicht enthalten sind. Bei einer Abweichung von Detektion-Signatur und Referenz-Signatur wird auf das Vorhandensein von Luftblasen geschlossen.

Der Erfindung liegt daher die Aufgabe zugrunde, ein Verfahren zum Überwachen einer strömenden Flüssigkeit auf das Vorhandensein von Luft anzugeben, das auch die Erkennung von relativ kleinen Luftblasen, insbesondere Mikroblasen zur Detektion unterschiedlicher Störfälle mit hoher Sicherheit erlaubt. Eine weitere Aufgabe der Erfindung ist, ein Verfahren zur extrakorporalen Blutbehandlung mit einem extrakorporalen Blutkreislauf anzugeben, mit dem sich mit hoher Sicherheit auch relativ kleine Luftblasen, insbesondere Mikroblasen im extrakorporalen Kreislauf erkennen lassen. Die Lösung dieser Aufgabe erfolgt erfindungsgemäß mit den Merkmalen der Patentansprüche 1 und 7.

Darüber hinaus liegt der Erfindung die Aufgabe zugrunde, eine Vorrichtung zum Überwachen einer strömenden Flüssigkeit auf das Vorhandensein von relativ kleinen Luftblasen, insbesondere Mikroblasen, und eine extrakorporale Blutbehandlungsvorrichtung mit einer derartigen Überwachungsvorrichtung bereitzustellen. Die Lösung dieser Aufgaben erfolgt mit den Merkmalen der Ansprüche 8 und 14.

Das Funktionsprinzip des erfindungsgemäßen Verfahrens und der erfindungsgemäßen Vorrichtung beruht darauf, dass eine Folge von Signalimpulsen oder ein kontinuierliches Signal in die strömende Flüssigkeit eingekoppelt und die aus der strömenden Flüssigkeit austretenden Signalimpulse bzw. das kontinuierliche Signal empfangen werden, wobei aus den empfangenen Signalimpulsen oder dem empfangenen kontinuierlichen Signal ein Signalmuster extrahiert wird, das für den zeitlichen Verlauf der empfangenen Signalimpulse oder des kontinuierlichen Signals in einem vorgegebenen Zeitintervall charakteristisch ist. Dieses charakteristische Signalmuster wird mit einem charakteristischen Referenzmuster verglichen, das für eine luftfreie Flüssigkeit charakteristisch ist, wobei auf das Vorhandensein von Luft dann geschlossen wird, wenn das charakteristische Signalmuster von dem charakteristischen Referenzmuster um ein vorgegebenes Maß abweicht. Grundsätzlich ist es möglich, alle aus der Mustererkennung bekannten Verfahren für die Auswertung des Empfangssignals heranzuziehen.

Für den Vergleich von Signalmuster und Referenzmuster ist unerheblich, wie die Signalimpulse oder das kontinuierliche Signal physikalisch realisiert werden. Vorzugsweise sind die Signalimpulse Ultraschall-Impulse bzw. das kontinuierliche Signal ein Ultraschallsignal. Die Signale können aber auch elektrische Felder oder elektromagnetische Strahlung sein. Allein entscheidend ist, dass die Signale durch das Vorhandensein von Luft in der strömenden Flüssigkeit verändert werden.

In einem definierten Zeitintervall $\Delta t$ durchlaufen die Messstrecke $\Delta N$ Mikroblasen, die eine kontinuierliche Volumenverteilung aufweisen. Das Vorhandensein von Mikroblasen führt zu einer zeitlichen Veränderung des Empfangssignals, das als kontinuierliches Signal $x(t)$ oder als kontinuierliche Folge von diskreten Werten $x$; (ll) vorliegen kann. Der zeitliche Signalverlauf des Empfangssignals stellt das charakteristische Signalmuster $E_{mess,\Delta N}$ dar.

Wenn keine Mikroblasen vorhanden sind, zeigt das Empfangssignal ein charakteristisches Signalmuster, das sich deutlich von dem charakteristischen Signalmuster des Empfangssignals unterscheidet, wenn Mikroblasen vorhanden sind. Das charakteristische Signalmuster wird nur von den Änderungen der Umgebungseinflüsse bestimmt, zu denen beispielsweise Störungen im Sender oder Empfänger oder Fluktuationen in der Dichte der strömenden Flüssigkeit zählen.

Das charakteristische Signalmuster stellt im Allgemeinen ein verrauschtes Signal dar. Mit zunehmender Luftinfusionsrate steigt die Anzahl $\Delta N$ der Mikroblasen, wobei sich die Verteilung der Mikroblasen in der Regel zu größeren Volumina verändert. Dies führt zu häufigeren Signaländerungen mit zum Teil höheren Änderungen der Signalamplituden. Das empfangene Muster ändert sich von einem luftfreien Muster $E_{mess,0}$ (charakteristisches Signalmuster) zu einem luftbeladenen Muster $E_{mess,\Delta N}$ (charakteristisches Signalmuster).

Das charakteristische Signalmuster $E_{mess,0}$ wird als charakteristisches Referenzmuster $E_{ref,O}$, vorzugsweise als Typmuster, festgelegt. Es ist auch möglich, das charakteristische Referenzmuster im Betrieb zyklisch neu zu bestimmen, wenn sichergestellt werden kann, dass sich keine Luftblasen in der Messstrecke befinden.

Wenn sich das charakteristische Signalmuster von dem charakteristischen Referenzmuster signifikant unterscheidet, kann ein Alarmzustand erzeugt werden. Dadurch kann die größtmögliche Empfindlichkeit für die Erkennung von Mikroblasen realisiert werden. Wenn systembedingt eine genügende Trennschärfe zwischen dem luftfreien Zustand und einem den Patienten gefährdenden Zustand gegeben ist, kann auch erst ab Erreichen eines vorgegebenen Grenzwertes ein Alarmzustand ausgelöst werden.

Neben dem luftfreien charakteristischen Signalmuster $E_{mess,0}$ als charakteristisches Referenzmuster $E_{ref,0}$ können noch weitere Referenzmuster $E_{ref,\Delta N}$ mit definierten Lufteinträgen verwendet werden, um zwischen verschiedenen Ereignissen unterscheiden zu können. Zum Beispiel kann ein Referenzmuster verwendet werden, das für eine einzelne Mikroblase charakteristisch ist, oder ein für die Überlagerung von n Mikroblasen charakteristisches Referenzmuster. Dadurch besteht die Möglichkeit, das Volumen der eingeschlossenen Luft als absolute Größe zu bestimmen.

Auch kann ein Referenzmuster verwendet werden, das einen Lufteintrag definiert, bei dem der Grenzwert für eine Gefährdung erreicht wird.

Das vorgegebene Zeitintervall, in dem der zeitliche Verlauf der empfangenen Signalimpulse oder des kontinuierlichen Signals bewertet wird, kann so klein gewählt werden dass die relativ langsamen Änderungen aufgrund der Umgebungseinflüsse als quasi konstant betrachtet werden können. Wenn dies der Fall ist, kann auf eine Kompensation der Änderungen aufgrund der Umgebungseinflüsse grundsätzlich verzichtet werden.

Es ist von Vorteil, wenn das Zeitintervall $\Delta t$ an die Größenordnung der Laufzeit der Mikroblasen durch die Messstrecke angepasst wird. Vorteilhaft ist, wenn das Zeitintervall $\Delta t$ in Abhängigkeit von dem Blutfluss vorgegeben wird.

Bei dem erfindungsgemäßen Verfahren oder der Vorrichtung wird der zeitliche Verlauf von Signal- und Referenzmuster nicht direkt miteinander verglichen. Im Allgemeinen ist es ausreichend, aus dem Signalmuster und dem Referenzmuster ein oder mehrere statistische Kenngrößen zu ermitteln, die für den zeitlichen Verlauf repräsentativ sind. Diese statistischen Kenngrößen können dann miteinander verglichen werden. Für das charakteristische Referenzmuster genügt im Allgemeinen ein oder mehrere definierte Kenngrößen.

Aufgrund der Natur eines Mikroblasenstromes kann selbst bei einem konstanten Lufteintrag nicht exakt vorhergesagt werden, wie viele Mikroblasen $\Delta N$ die Messstrecke im Zeitintervall $\Delta t$ passieren werden und welche Häufigkeitsverteilung die Volumina dieser $\Delta N$ Mikroblasen haben werden. Das charakteristische Signalmuster kann aber als das Ergebnis eines stochastischen Prozesses aufgefasst und mit den Algorithmen der statistischen Analyse von Zeitfolgen in vorteilhafter Weise mit verhältnismäßig geringem Aufwand bewertet werden. Die Bewertung kann auf der Grundlage einer Häufigkeitsverteilung im Zeitbereich oder auf der Grundlage einer Spektralanalyse stochastischer Prozesse erfolgen.

Bei der Bewertung der Häufigkeitsverteilung im Zeitbereich wird das Empfangssignal als eindimensionale Zufallsgröße X aufgefasst, wobei ihre Realisierungen x einen diskreten oder stetigen Wertebereich umfassen können. Die statistischen Eigenschaften der Zufallsgröße werden vollständig durch ihre Verteilungsfunktion F(x) bzw. durch die kontinuierliche Verteilungsdichtefunktion f(x) oder durch die diskreten Wahrscheinlichkeiten $w_i$ beschrieben.

Um den Aufwand zu reduzieren, ist es vorteilhaft, nicht die vollständigen Verteilungsfunktionen F(x) zu bestimmen und mit einander zu vergleichen, sondern sich auf ein oder mehrere charakteristische Merkmale zu beschränken. Die Definition dieser aussagekräftigen Kennwerte erfolgt in der Regel als Erwartungswerte einer Funktion g(x) gemäß:

$$E(g(X)) = \int_{-\infty}^{\infty} g(x) f(x) dx \qquad (1.1a)$$

$$E(g(X)) = \sum_i g(x_i) w_i \qquad (1.1b)$$

Bei einer stetigen Zufallsgröße X gilt Gl. (1.1a) und bei einer diskreten Zufallsgröße X gilt Gl. (1.1b), wobei $w_i$ die Wahrscheinlichkeit der Realisierung von $x_i$ ist.

Eine weitere vorteilhafte Vereinfachung ist es, wenn der Mittelwert E(X) in den folgenden Gleichungen nicht aus den Daten des aktuellen Zeitintervalls $\Delta t$ berechnet wird, sondern der von vorherigen Intervallen verwendet wird. Dies verursacht in der Regel zusätzliche Fehler von akzeptabler Größe, wenn das Zeitintervall $\Delta t$ so klein gewählt wird, dass die typische zeitliche Änderung des Mittelwertes vernachlässigbar ist.

Vorteilhafte Erwartungswerte sind:

1. Mittelwert:

$$g(X) = X \qquad (1.2)$$

2. Streuung oder Varianz $\sigma^2$ bzw. Standardabweichung $\sigma$:

$$g(X) = (X - E(X))^2 \qquad (1.3)$$

3. Momente k-ter Ordnung

$$g(X) = X^k \qquad (1.4)$$

4. Absolute Momente k-ter Ordnung

$$g(X) = |X|^k \qquad (1.5)$$

5. Momente k-ter Ordnung bezüglich c

$$g(X) = (X - c)^k \qquad (1.6)$$

6. Zentrierte Momente k-ter Ordnung

$$g(X) = (X - E(X))^k \qquad (1.7)$$

7. Absolute zentrierte Momente k-ter Ordnung

$$g(X) = |X - E(X)|^k \qquad (1.8)$$

8. Schiefe der Verteilung von X

9. Exzess der Verteilung von X

10. Autokorrelationsfunktion
Der Korrelationsmittelwert bezieht sich auf einen zufälligen Prozess X.
Für zweidimensionale Zufallsgrößen sind die folgenden Kennwerte von Bedeutung. Ein Referenz-Muster $E_{ref,\Delta N}$ kann z. B. als Realisierung der Zufallsgröße $X_1$ und ein aktuell gemessenes Muster $E_{mess}$ kann als Realisierung der Zufallsgröße $X_2$ aufgefasst werden.

11. Kovarianz

$$g(X) = (X_1 - E(X_1))(X_2 - E(X_2)) \qquad (1.9)$$

12. Korrelationskoeffizient

$$g(X) = (X_1 - E(X_1)) / \sigma_1 (X_2 - E(X_2)) / \sigma_2 \qquad (1.10)$$

13. Kreuzkorrelationsfunktion

Der Korrelationsmittelwert bezieht sich auf zwei verschiedene zufällige Prozesse $X_1$, $X_2$.

Neben der Bewertung der Häufigkeitsverteilung im Zeitbereich kann auch die Spektralanalyse zur Ermittlung von einer oder mehreren Kenngrößen aus den Signalmustern herangezogen werden. Das Ziel der Spektralanalyse (Fourier- Analyse) besteht darin, eine komplexe Zeitfolge mit zyklischen Komponenten in wenige zugrundeliegende harmonische Funktionen mit bestimmter Frequenz zu zerlegen, wobei diese Zeitfolge als Realisierung einer eindimensionalen Zufallsgröße X aufgefasst werden kann. Bei der Auswertung des Empfangssignals sind im Spektrum nur die Frequenzbereiche relevant, die durch Mikroblasen verändert werden. Dabei können die relativ hoch- frequenten Signalanteile aufgrund von empfangen Störungen oder durch elektronisches Rauschen durch Filterfunktionen eliminiert werden. Die relativ nieder- frequenten Signalanteile aufgrund der Änderung von Umgebungseinflüssen können ebenso ausgeblendet werden.

Vorteilhafte Kenngrößen sind:

1. Periodogramm (Summe der Amplitudenquadrate bestimmter Frequenzen im Sinne eines Linienspektrums)

2. Spektraldichtefunktion (Energiedichtespektrum oder Leistungsdichtespektrum für bestimmte Frequenzen)

3. Anwendung von Filtern (Glättung innerhalb von Spektralfenstern)

Bei dem Vergleich der Signalmuster kann es von Vorteil sein, mehrere Kenngrößen miteinander zu kombinieren, um die Trennschärfe für die Detektion von Mikroblasen zu erhöhen. Beispielsweise ist es möglich, auf einen Störfall dann zu schließen, wenn zwei Kenngrößen eine bestimmte Veränderung erfahren haben und eine dritte Kenngröße sich statistisch nicht signifikant verändert hat

Als besonders vorteilhaft hat sich als statistische Kenngröße die Varianz erwiesen (Standardabweichung, Streumaß). Die Varianz ist ein Maß dafür, wie die einzelnen Daten um den Mittelwert verteilt sind, d. h. wie stark die Daten um den Mittelwert streuen. Da die Varianz eine vom Mittelwert bereinigte Größe ist, werden die Umgebungseinflüsse eliminiert, da die Änderung der absoluten Signalhöhe, beispielsweise des Signalmittelwertes, im Zeitintervall $\Delta t$ nicht als eine Änderung des Musters bewertet wird.

Bei einem weiteren bevorzugten Ausführungsbeispiel des erfindungsgemäßen Verfahrens und der Vorrichtung werden charakteristische Signalmuster in einer Folge von Zeitintervallen fortlaufend ermittelt und jeweils mit einem oder mehreren Referenzmustern verglichen. Ein Alarm kann schon dann gegeben werden, wenn eines der charakteristischen Signalmuster von dem charakteristischen Referenzmuster um ein vorgegebenes Maß abweicht Es ist aber auch möglich Alarm erst dann zu geben, wenn in einer bestimmten Anzahl von Zeitintervallen eine Abweichung um ein vorgegebenes Maß festgestellt wird. Die Anzahl der Ereignisse, bei der eine Abweichung festgestellt wird, wird während der Blutbehandlung gezählt. Die Anzahl der Ereignisse ist dabei ein Maß für das Volumen der insgesamt in der Flüssigkeit eingeschlossenen Luft.

Eine weitere bevorzugte Ausführungsform sieht vor, eine Folge von Signalimpulsen anstelle eines kontinuierlichen Signals in die strömende Flüssigkeit einzukoppeln, wobei als charakteristisches Signalmuster die maximalen Signalamplituden der empfangenen Signalimpulse in einem vorgegebenen Zeitraum ermittelt werden. Anstelle der maximalen Signalamplitude können aber auch alle mit der Signalamplitude korrelierenden Größen bestimmt werden.

Im Folgenden wird ein Ausführungsbeispiel der Erfindung unter Bezugnahme auf die Zeichnungen näher erläutert.

Es zeigen:

Figur 1   die wesentlichen Komponenten einer Blutbehandlungsvorrichtung mit einem extrakorporalen Blutkreislauf in stark vereinfachter schematischer Darstellung zusammen mit einer Vorrichtung zum Überwachen des im Blutkreislauf strömenden Blutes auf das Vorhandensein von Luft,

Figur 2   die wesentlichen Komponenten eines Ausführungsbeispiels der Vorrichtung zum Überwachen des Blutes auf das Vorhandensein von Luft in schematischer Darstellung,

Figur 3   den zeitlichen Verlauf des charakteristischen Referenzmusters, wenn keine Luftblasen in der strömenden Flüssigkeit vorhanden sind,

Figur 4a   den zeitlichen Verlauf des charakteristischen Signalmusters, wenn viele Mikroblasen in der Flüssigkeit vorhanden sind,

Figur 4b   einen Ausschnitt des Signalmusters von Figur 4a in vergrößerter Darstellung,

Figur 5a     den zeitlichen Verlauf des charakteristischen Signalmusters, wenn wenige Mikroblasen in der Flüssigkeit vorhanden sind und

Figur 5b     einen Ausschnitt des Signalmusters von Figur 5a in vergrößerter Darstellung.

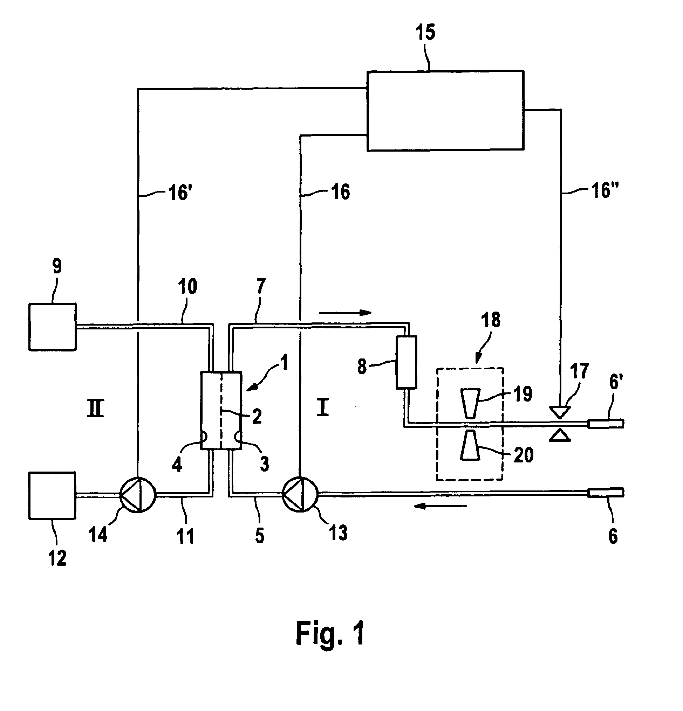

Figur 1 zeigt die wesentlichen Komponenten der Blutbehandlungsvorrichtung zusammen mit der Überwachungsvorrichtung. Die Blutbehandlungsvorrichtung, beispielsweise eine Hämodialysevorrichtung, weist einen Dialysator 1 auf, der durch eine semipermeable Membran 2 in eine Blutkammer 3 und eine Dialysierflüssigkeitskammer 4 getrennt ist. Der Einlass der Blutkammer ist mit einem Ende der Blutzuführleitung 5 verbunden, während der Auslass der Blutkammer 3 mit dem einen Ende einer Blutabführleitung 7 verbunden ist, in die eine Tropfkammer 8 geschaltet ist. Die anderen Enden der Blutzuführ- und -abführleitung 5, 7 sind mit einer arteriellen bzw. venösen Nadel 6, 6' verbunden. Zwischen Tropfkammer 8 und venöser Nadel 6' ist eine elektromagnetisch betätigbare venöse Schlauchklemme 17 an der Blutabführleitung 7 angeordnet. Die Blutzuführ- und -abführleitung 5, 7 stellen zusammen mit der Blutkammer 3 des Dialysators 1 den extrakorporalen Blutkreislauf I der Hämodialysevorrichtung dar.

Das Dialysierflüssigkeitssystem II der Dialysevorrichtung umfasst eine Einrichtung 9 zur Aufbereitung der Dialysierflüssigkeit, von der eine Dialysierflüssigkeitszuführleitung 10 abgeht, die zu der Dialysierflüssigkeitskammer 4 des Dialysators 1 führt. Von der Dialysierflüssigkeitskammer 4 geht eine Dialysierflüssigkeitsabführleitung 11 ab, die zu einem Auslass 12 führt.

In der Blutzuführleitung 5 ist eine Blutpumpe 13 angeordnet, während in der Dialysierflüssigkeitsabführleitung 11 eine Dialysierflüssigkeitspumpe 14 angeordnet ist. Blutpumpe 13 und Dialysierflüssigkeitspumpe 14 fördern während der Blutbehandlung Blut im extrakorporalen Blutkreislauf I bzw. Dialysierflüssigkeit im Dialysierflüssigkeitssystem II.

Die Hämodialysevorrichtung umfasst eine zentrale Steuereinheit 15, die über Steuerleitungen 16, 16', 16'' mit der Blutpumpe 13 bzw. Dialysierflüssigkeitspumpe 14 bzw. Schlauchklemme 17 verbunden ist.

Darüber hinaus verfügt die Hämodialysevorrichtung über eine Vorrichtung zum Überwachen des im extrakorporalen Blutkreislaufs I strömenden Blutes auf das Vorhandensein von Luft. Diese Überwachungsvorrichtung ist bei der beschriebenen Vorrichtung Bestandteil der Hämodialysevorrichtung, sie kann aber auch eine separate Baugruppe bilden.

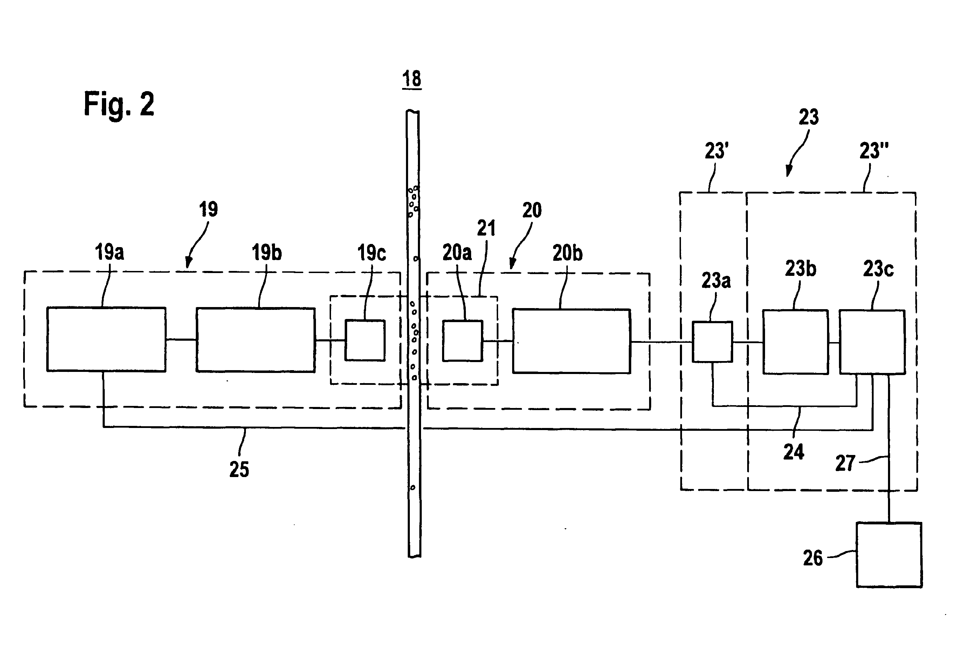

In Figur 1 ist die Überwachungsvorrichtung 18 nur schematisch dargestellt. Die einzelnen Komponenten der Überwachungsvorrichtung zeigt Figur 2.

Die Überwachungsvorrichtung 18 verfügt über einen Ultraschall-Sender 19 und einen Ultraschall-Empfänger 20. Der Ultraschallsender 19 weist einen Impulsgenerator 19a und eine Leistungsstufe 19b sowie einen Ultraschallwandler 19c auf, während der Ultraschall-Empfänger ein Ultraschall-Wandler 20a und einen Signalverstärker 20b aufweist. Der Ultraschallwandler 19c des Senders 19 und der Ultraschall-Wandler 20a des Empfängers 20 sind auf einem Sensorträger 21 parallel zueinander zu beiden Seiten der Blutabführleitung 7 stromab der Tropfkammer 8 und stromauf der Schlauchklemme 17 derart angeordnet, dass das in der Blutabführleitung 7 strömende Blut orthogonal mit Ultraschall durchstrahlt wird. Die beiden Ultraschall-Wandler 19c und 20a können piezoelektrische Keramikscheiben sein. Der Zwischenraum zwischen den beiden Ultraschallwandlern bildet eine akustische Messstrecke.

Der Ultraschall-Sender 19 arbeitet im Impulsbetrieb. Dazu wird der Impulsgenerator 19a über einen für die Ablaufsteuerung und Signalanalyse verantwortlichen Mikrocontroller 23c, der mit dem Impulsgenerator 19a über eine Signaleitung 25 verbunden ist, zyklisch angesteuert. Der Impulsgenerator 19a erzeugt daraufhin elektrische Signale, die über die Leistungsstufe 19b dem Ultraschall-Wandler 19c zugeführt werden.

Der Ultraschall-Wandler 19c des Empfängers 20 wandelt die Ultraschallsignale wieder in elektrische Signale um, die dem Signalverstärker 20b zugeführt werden. Der Signalverstärker 20b filtert und bereitet das elektrische Signal so auf, das es einer Auswerteinheit 23 zugeführt werden kann.

Die Auswerteinheit 23 verfügt über Mittel 23' zum Extrahieren eines Signalmusters, das für den zeitlichen Verlauf der empfangenen Ultraschallimpulse charakteristisch ist und Mittel 23'' zum Vergleichen des charakteristischen Signalmusters mit einem Referenzmuster.

Die Mittel 23' zum Extrahieren des Signalmusters weisen einen Peakdetektor 23a auf, der als Maß für die Pegel der vom Ultraschallwandler 20a empfangenen Ultraschallimpulse die maximale Signalamplitude der Impulse bestimmt, während die Mittel 23'' zum Vergleichen des charakteristischen Signalmusters mit einer Referenzmuster einen A/D-Wandler 23b und den Mikrokontroller 23c aufweisen.

Der vom Peakdetektor 23a gelieferte analoge Spannungswert wird von dem A/D-Wandler 23b in einen digitalen Wert zur weiteren Signalverarbeitung überführt. Vor jeder Abgabe eines Ultraschallimpulses über den Ultraschallwandler 19c wird der Peakdetektor 23a von dem Mikrocontroller 23c zurückgesetzt, der über eine Signalleitung 24 mit dem Peakdetektor verbunden ist. Am Ausgang des Peakdetektors 23a liegt also ein Signalmuster an, das für den zeitlichen Verlauf der empfangenen Ultraschallimpulse in einem vorgegebenen Zeitintervall $\Delta t$ charakteristisch ist. Das vorgegebene Zeitintervall $\Delta t$ umfasst bei dem Ausführungsbeispiel insgesamt m = 128 Werte, wobei jeder Wert die maximale Amplitude des empfangenen Ultraschallimpulses darstellt.

Aufgrund der konstanten Erregung des Ultraschallwandlers 19c des Senders 19 zeigen sich Veränderungen der akustischen Eigenschaften der Messstrecke in der Größe der maximalen Amplitude des vom Peakdetektor 23a gelieferten Analogsignals bzw. der Größe des am A/D-Wandler anliegenden Digitalsignals.

Die akustischen Eigenschaften der Messstrecke ändern sich dann, wenn ungelöste Luft beispielsweise in Form von einzelnen winzigen Luftblasen, d. h. Mikroblasen, in dem durch die Blutabführleitung 7 strömenden Blut vorhanden sind. Die Mikroblasen in der akustischen Messstrecke führen zu einer Dämpfung der Ultraschallimpulse und somit zu einem Einbruch der maximalen Signalamplitude. Der über die Zeit messbare Verlauf der maximalen Amplitude erfährt also eine Modulation entsprechend der gerade wirksamen Dämpfung der akustischen Messstrecke.

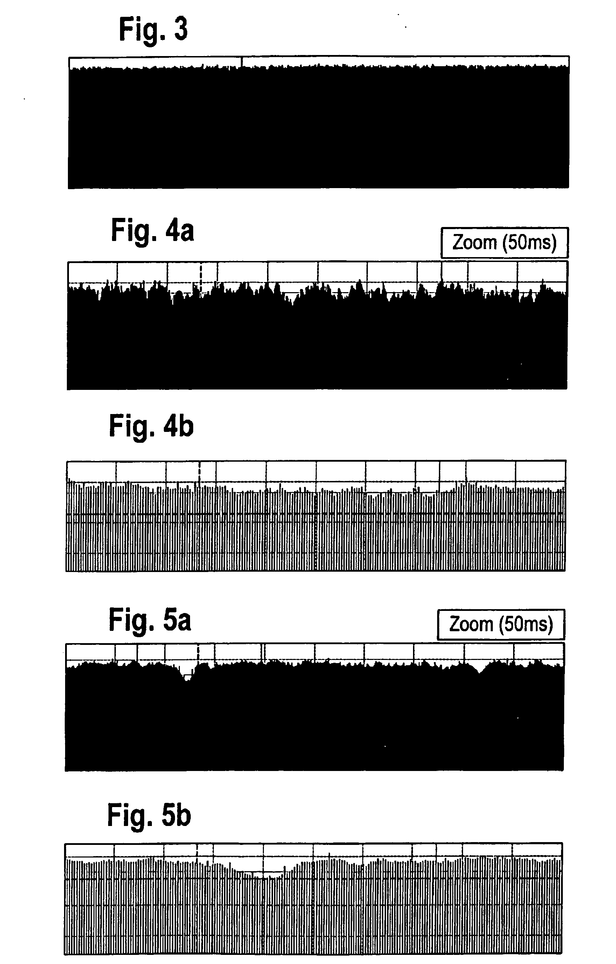

Wenn keine Mikroblasen in dem Blut vorhanden sind, können signifikante Einbrüche der Signalamplitude nicht beobachtet werden. Figur 3 zeigt ein derartiges Signalmuster, das als charakteristisches Referenzmuster herangezogen wird. Es sind nur winzige Signaländerungen (Rauschen) zu erkennen, die eine relativ hohe Frequenz haben.

Die Figuren 4a und 4b zeigen ein Signalmuster, das für das Vorhandensein von einer Vielzahl von Mikroblasen charakteristisch ist. Deutlich sind die Einbrüche der Signalamplitude zu erkennen. Das in Figur 4a gezeigte Signalmuster umfasst eine Vielzahl von aufeinanderfolgenden Zeitintervallen $\Delta t$. Der zeitliche Verlauf des Signalmusters für eines der Zeitintervalle $\Delta t$ ist in Figur 4b vergrößert dargestellt. Typisch sind die relativ großen Änderungen des Signals, die aber eine relativ niedrige Frequenz haben. Das Zeitintervall $\Delta t$ umfasst m=128 Werte.

Die Figuren 5a und 5b zeigen Signalmuster, die für das Vorhandensein von wenigen Mikroblasen charakteristisch sind. Der Vergleich der Signalmuster von Figur 4a, 4b und Figur 5a und 5b zeigt, dass in dem Signalmuster von Figur 5a, 5b deutlich weniger Einbrüche der Signalamplitude zu erkennen sind.

Um das charakteristische Signalmuster mit dem Referenzmuster vergleichen zu können, wird in der Auswerteinheit 23, deren Bestandteil der Mikrokontroller 23c ist, eine charakteristische statistische Kenngröße aus dem Signalmuster für jedes der aufeinanderfolgenden Zeitintervalle bestimmt. Die charakteristische statistische Kenngröße ist bei dem Ausführungsbeispiel die Varianz $\sigma^2$ des Signalmusters. Da die Varianz ein Merkmal ist, bei dem die Messwerte definitionsgemäß vom Mittelwert bereinigt sind, wird eine Unterdrückung des Einflusses der Umgebungsbedingungen erreicht.

Die Berechnung der Varianz erfolgt nach den Gleichungen 1.1b und 1.3. Für die Wahrscheinlichkeiten $w_i$ in Gleichung 1.1b wird ein konstanter Wert eingesetzt. Da die Datenbasis eine Stichprobe und keine Grundgesamtheit ist, ergibt sich aus der Normierungsbedingung und der Forderung der Erwartungstreue dieser Wert zu $w_i=1/(m-1)$, wobei das Muster m=128 Werte umfasst.

Der Mittelwert E(x) wird dabei in den Gleichungen nicht aus den Daten des Zeitintervalls $\Delta t$ berechnet, in dem gerade die Auswertung erfolgt, sondern der in einem oder mehreren der vorausgegangenen Zeitintervalle ermittelte Mittelwert verwendet. Dies setzt aber voraus, dass das gewählte Zeitintervalls $\Delta t$ so klein ist, dass Änderungen des Mittelwertes zu vernachlässigen sind.

Bei dem Ausführungsbeispiel wird darauf verzichtet, auch aus dem charakteristischen Referenzmuster von Figur 3 die Varianz zu berechnen. Es genügt, als Varianz für das Referenzmuster einen Grenzwert vorzugeben, bei dem angenommen wird, dass bei dessen Überschreiten eine Gefährdung für den Patienten gegeben ist.

In der Auswerteinheit 23 wird nun die Varianz $\sigma^2$ des charakteristischen Signalmusters mit dem vorgegebenen Grenzwert verglichen. Wenn die Varianz $\sigma^2$ größer als der Grenzwert ist, wird darauf geschlossen, dass in dem vorgegebenen Zeitintervall $\Delta t$ Mikroblasen vorhanden sind. Ein Vergleich mit mehreren Grenzwerten erlaubt die Unterscheidung zwischen dem Vorliegen von wenigen oder vielen Mikroblasen.

Für den Fall, dass ein für den Patienten gefährliches Volumen an Mikroblasen in einem vorgegebenen Zeitintervall detektiert wird, erzeugt die Auswerteinheit 23 ein Alarmsignal, das eine Alarmeinheit 26 empfängt, die über eine Steuerleitung 27 mit der Auswerteinheit 23 verbunden ist. Die Alarmeinheit 26 erzeugt dann einen akustischen und/oder optischen Alarm. Gleichzeitig erzeugt die Alarmeinheit 26 ein Steuersignal, das über eine nicht dargestellte Signalleitung an der Steuereinheit 15 der Dialysevorrichtung anliegt. Wenn dieses Steuersignal erzeugt wird, unterbricht die Steuereinheit 15 die Dialysebehandlung, indem sie die Blutpumpe 13 anhält und die Schlauchklemme 17 zum Abklemmen der Blutabführleitung 7 betätigt.

Eine alternative Ausführungsform sieht vor, dass nur dann Alarm gegeben wird, wenn in einer Mehrzahl von Zeitintervallen Mikroblasen erkannt werden.

Für den Fall, dass die Auswerteinheit 23 Mikroblasen in einem der Zeitintervalle $\Delta t$ erkennt, erzeugt die Auswerteinheit 23 ein Zählsignal. Während der Blutbehandlung oder während einer kontinuierlichen oder diskontinuierlichen Folge von Zeitabschnitten der Dialysebehandlung werden in den aufeinanderfolgenden Messungen die Anzahl der von der Auswerteinheit 23 erzeugten Zählsignale gezählt. Die Anzahl der Zählsignale wird während der Dialysebehandlung fortlaufend mit einem weiteren vorgegebenen Grenzwert verglichen. Wenn der Zählstand den Grenzwert erreicht, erzeugt die Auswerteinheit 23 wieder ein Alarmsignal, so dass die Alarmeinheit 26 einen akustischen und/oder optischen Alarm gibt und die Steuereinheit 15 die Blutbehandlung unterbricht. In diesem Fall wird davon ausgegangen, dass das Gesamtvolumen der im Blut eingeschlossenen Luft eine Gefahr für den Patienten darstellt.

**EP 1 886 130 B1**

**Patentansprüche**

1. Verfahren zum Überwachen eines strömenden Mediums auf das Vorhandensein von Luft mit folgenden Verfahrensschritten:

   Einkoppeln einer Folge von Signalimpulsen oder eines kontinuierlichen Signals in das strömende Medium, Empfangen der aus dem strömenden Medium austretenden Signalimpulse oder des kontinuierlichen Signals, Extrahieren eines Signalmusters, das für den zeitlichen Verlauf der empfangenden Signalimpulse oder des kontinuierlichen Signals in einem vorgegebenen Zeitintervall charakteristisch ist und
   Vergleichen des charakteristischen Signalmusters mit einem oder mehreren charakteristischen Referenzmustern, wobei auf das Vorhandensein von Luft geschlossen wird, wenn das charakteristische Signalmuster von mindestens einem charakteristischen Referenzmuster um ein vorgegebenes Maß abweicht,
   **dadurch gekennzeichnet, dass**
   zum Vergleichen des charakteristischen Signalmusters mit einem oder mehreren charakteristischen Referenzmustern aus dem charakteristischen Signalmuster ein oder mehrere statistische Kenngrößen ermittelt werden, die mit einer oder mehreren statistischen Referenzgrößen verglichen werden.

2. Verfahren nach Anspruch 1, **dadurch gekennzeichnet, dass** charakteristische Signalmuster in einer Folge von Zeitintervallen fortlaufend ermittelt und jeweils mit einem oder mehreren Referenzmustern verglichen werden.

3. Verfahren nach Anspruch 1 oder 2, **dadurch gekennzeichnet, dass** die statistische Kenngröße die Varianz ist.

4. Verfahren nach einem der Ansprüche 1 bis 3, **dadurch gekennzeichnet, dass** charakteristische Signalmuster in einer Folge von Zeitintervallen fortlaufend ermittelt und jeweils mit einem oder mehreren Referenzmustern verglichen werden, wobei zur Berechnung der Varianz als statistische Kenngröße der in einem oder mehreren der vorausgehenden Zeitintervalle berechnete Mittelwert herangezogen wird.

5. Verfahren nach einem der Ansprüche 1 bis 4, **dadurch gekennzeichnet, dass** die maximalen Signalamplituden der empfangenen Signalimpulse ermittelt werden, wobei das charakteristische Signalmuster die in einem vorgegebenen Zeitintervall auftretende Folge der maximalen Amplituden der Signalimpulse ist.

6. Verfahren nach einem Ansprüche 1 bis 5, **dadurch gekennzeichnet, dass** die Folge von Signalimpulsen oder das kontinuierliche Signal eine Folge von Ultraschallimpulsen bzw. ein kontinuierliches Ultraschallsignal ist.

7. Verfahren zur extrakorporalen Blutbehandlung mit einem extrakorporalen Blutkreislauf, wobei das Vorhandensein von Luft im extrakorporalen Blutkreislauf mit einem Verfahren nach einem der Ansprüche 1 bis 6 überwacht wird.

8. Vorrichtung zum Überwachen eines strömenden Mediums auf das Vorhandensein von Luft mit
   Mitteln (19) zum Einkoppeln einer Folge von Signalimpulsen und eines kontinuierlichen Signals in das strömende Medium,
   Mitteln (20) zum Empfangen der aus der strömenden Flüssigkeit austretenden Signalimpulse oder des kontinuierlichen Signals und
   einer Auswerteinheit (23), die aufweist:

   Mittel (23') zum Extrahieren eines Signalmusters, das für den zeitlichen Verlauf der empfangenen Signalimpulse oder des kontinuierlichen Signals in einem vorgegebenen Zeitintervall charakteristisch ist und
   Mittel (23") zum Vergleichen des charakteristischen Signalmusters mit einem oder mehreren charakteristischen Referenzmustern, wobei auf das Vorhandensein von Luft geschlossen wird, wenn das charakteristische Signalmuster von mindestens einem charakteristischen Referenzmuster um ein vorgegebenes Maß abweicht,
   **dadurch gekennzeichnet, dass** die Mittel (23") zum Vergleichen des charakteristischen Signalmusters mit einem oder mehreren charakteristischen Referenzmustern derart ausgebildet sind, dass aus dem charakteristischen Signalmuster ein oder mehrere statistische Kenngrößen ermittelt werden, die mit einer oder mehreren statistischen Referenzgrößen verglichen werden.

9. Vorrichtung nach Anspruch 8, **dadurch gekennzeichnet, dass** die Mittel (23') zum Extrahieren eines Signalmusters derart ausgebildet sind, dass charakteristische Signalmuster in einer Folge von Zeitintervallen fortlaufend ermittelt und jeweils mit einem oder mehreren Referenzmustern verglichen werden.

**10.** Vorrichtung nach Anspruch 8 oder 9, **dadurch gekennzeichnet, dass** die Mittel (23 ") zum Vergleichen des charakteristischen Signalmuster mit einem oder mehreren charakteristischen Referenzmustern derart ausgebildet sind, dass als statistische Kenngröße die Varianz berechnet wird.

**11.** Vorrichtung nach einem der Ansprüche 8 bis 10, **dadurch gekennzeichnet, dass** die Mittel (23") zum Vergleichen des charakteristischen Signalmuster mit einem oder mehreren charakteristischen Referenzmustern derart ausgebildet sind, dass charakteristische Signalmuster in einer Folge von Zeitintervallen fortlaufend ermittelt und jeweils mit einem oder mehreren Referenzmustern verglichen werden, wobei zur Berechnung der Varianz als statistische Kenngröße der in einem oder mehreren der vorausgehenden Zeitintervalle berechnete Mittelwert herangezogen wird.

**12.** Vorrichtung nach einem der Ansprüche 8 bis 11, **dadurch gekennzeichnet, dass** die Mittel (23') zum Extrahieren eines Signalmusters derart ausgebildet sind, dass als charakteristisches Signalmuster die in einem vorgegebenem Zeitintervall auftretende Folge der maximalen Amplituden der Signalimpulse extrahiert wird.

**13.** Vorrichtung nach einem der Ansprüche 8 bis 12, **dadurch gekennzeichnet, dass** die Mittel zum Senden der Folge von Signalimpulsen oder des kontinuierlichen Signals ein Ultraschall-Sender (19), und die Mittel zum Empfangen der Ultraschallimpulse ein Ultraschall-Empfänger (20) sind.

**14.** Vorrichtung zur extrakorporalen Blutbehandlung mit einem extrakorporalen Blutkreislauf und einer Vorrichtung nach einem der Ansprüche 8 bis 13, wobei das Vorhandensein von Luft im extrakorporalen Blutkreislauf überwacht wird.

**Claims**

**1.** A method for monitoring a flowing medium for the presence of air, comprising the following method steps:

  injecting a sequence of signal pulses or a continuous signal into the flowing medium,
  receiving the signal pulses or the continuous signal leaving the flowing medium,
  extracting a signal pattern that is characteristic for the chronological progression of the received signal pulses or the continuous signal within a predetermined time interval, and
  comparing the characteristic signal pattern with one or a plurality of characteristic reference patterns, wherein the presence of air is concluded if the characteristic signal pattern deviates from at least one characteristic reference pattern by a predetermined amount,
  **characterized in that**
  for comparing the characteristic signal pattern with one or a plurality of characteristic reference patterns, one or a plurality of statistical variables are determined from the characteristic signal pattern, which statistical variables are compared with one or a plurality of statistical reference variables.

**2.** The method according to claim 1, **characterized in that** characteristic signal patterns are continuously determined in a sequence of time intervals and are compared in each case with one or a plurality of reference patterns.

**3.** The method according to claim or claim 2, **characterized in that** the statistical variable is variance.

**4.** The method according to any one of the claims 1 to 3, **characterized in that** characteristic signal patterns are continuously determined in a sequence of time intervals and are compared in each case with one or a plurality of reference patterns, wherein for calculating the variance as a statistical variable, the average value calculated in one or a plurality of the preceding time intervals is used.

**5.** The method according to any one of the claims 1 to 4, **characterized in that** the maximum signal amplitudes of the received signal pulses are determined, wherein the characteristic signal pattern is the sequence of the maximum signal pulse amplitudes occurring within a predefined time interval.

**6.** The method according to any one of the claims 1 to 5, **characterized in that** the sequence of signal pulses or the continuous signal is a sequence of ultrasound pulses or a continuous ultrasound signal.

**7.** A method for extracorporeal blood treatment using an extracorporeal blood circulation, wherein the presence of air in the extracorporeal blood circulation is monitored with a method according to any one of the claims 1 to 6.

8. A device for monitoring a flowing medium for the presence of air, comprising
means (19) for injecting a sequence of signal pulses and a continuous signal into the flowing medium,
means (20) for receiving the signal pulses or the continuous signal leaving the flowing medium, and
an evaluation unit (23), comprising:

means (23') for extracting a signal pattern that is characteristic for the chronological progression of the received signal pulses or the continuous signal within a predefined time interval, and
means (23") for comparing the characteristic signal pattern with one or a plurality of characteristic reference patterns, wherein the presence of air is concluded if the characteristic signal pattern deviates from at least one characteristic reference pattern by a predetermined amount,
**characterized in that** the means (23") for comparing the characteristic signal pattern with one or a plurality of characteristic reference patterns are configured such that from the characteristic signal pattern, one or a plurality of statistical variables are determined which are compared with one or a plurality of statistical reference variables.

9. The device according to claim 8, **characterized in that** the means (23') for extracting a signal pattern are configured such that characteristic signal patterns are continuously determined in a sequence of time intervals and are compared in each case with one or a plurality of reference patterns.

10. The device according to claim 8 or claim 9, **characterized in that** the means (23") for comparing the characteristic signal pattern with one or a plurality of characteristic reference patterns are configured such that variance is calculated as a statistical variable.

11. The device according to any one of the claims 8 to 10, **characterized in that** the means (23") for comparing the characteristic signal pattern with one or a plurality of characteristic reference patterns are configured such that characteristic signal patterns are continuously determined in a sequence of time intervals and are compared in each case with one or a plurality of reference patterns, wherein for calculating the variance as a statistical variable, the average value calculated in one or a plurality of the preceding time intervals is used.

12. The device according to any one of the claims 8 to 11, **characterized in that** the means (23') for extracting a signal pattern are configured such that as a characteristic signal pattern, the sequence of maximum signal pulse amplitudes occurring in a predetermined time interval is extracted.

13. The device according to any one of the claims 8 to 12, **characterized in that** the means for transmitting the sequence of signal pulses or the continuous signal is an ultrasound transmitter (19), and the means for receiving the ultrasound pulses is an ultrasound receiver (20).

14. A device for extracorporeal blood treatment using an extracorporeal blood circulation and a device according to any one of the claims 8 to 13, wherein the presence of air in the extracorporeal blood circulation is monitored.

**Revendications**

1. Procédé de surveillance de la présence d'air dans un fluide en écoulement avec les étapes suivantes :

introduction d'une série d'impulsions de signaux ou d'un signal continu dans le fluide en écoulement,
réception des impulsions de signaux ou du signal continu sortant du fluide en écoulement,
extraction d'un échantillon de signal qui est caractéristique du tracé temporel des impulsions de signaux ou du signal continu reçus dans un intervalle de temps prédéterminé et
comparaison de l'échantillon de signal caractéristique avec un ou plusieurs échantillons de référence caractéristiques, la présence d'air étant déterminée lorsque l'échantillon de signal caractéristique diffère d'au moins un échantillon de référence caractéristique d'un facteur prédéterminé,
**caractérisé en ce que**
pour la comparaison de l'échantillon de signal caractéristique avec un ou plusieurs échantillons de référence caractéristiques, une ou plusieurs grandeurs statistiques caractéristiques sont déterminées à partir de l'échantillon de signal caractéristique, qui sont comparées avec une ou plusieurs grandeurs statistiques de référence.

2. Procédé selon la revendication 1, **caractérisé en ce que** les échantillons de signaux caractéristiques sont déterminés en continu dans une série d'intervalles de temps et comparés chacun avec un ou plusieurs échantillons de référence.

**3.** Procédé selon la revendication 1 ou 2, **caractérisé en ce que** la grandeur statistique caractéristique est la variance.

**4.** Procédé selon l'une des revendications 1 à 3, **caractérisé en ce que** les échantillons de signaux caractéristiques sont déterminés en continu dans une série d'intervalles de temps et comparés chacun avec un ou plusieurs échantillons de référence, la valeur moyenne calculée dans un ou plusieurs intervalles de temps précédents étant utilisée pour calculer la variance en tant que grandeur statistique caractéristique.

**5.** Procédé selon l'une des revendications 1 à 4, **caractérisé en ce que** les amplitudes maximales des impulsions de signaux reçues sont déterminées, l'échantillon de signal caractéristique étant la série, apparaissant dans un intervalle de temps prédéterminé, des amplitudes maximales des impulsions de signaux.

**6.** Procédé selon l'une des revendications 1 à 5, **caractérisé en ce que** la série d'impulsions de signaux ou le signal continu est une série d'impulsions ultrasonores ou un signal ultrasonore continu.

**7.** Procédé pour le traitement extracorporel du sang avec une circulation sanguine extracorporelle, la présence d'air dans la circulation sanguine extracorporelle étant surveillée avec un procédé selon l'une des revendications 1 à 6.

**8.** Dispositif de surveillance de la présence d'air dans un fluide en écoulement avec :

des moyens (19) permettant d'introduire une série d'impulsions de signaux et d'un signal continu dans le fluide en écoulement,
des moyens (20) permettant de recevoir les impulsions de signaux ou du signal continu sortant du liquide en écoulement et
une unité d'analyse (23) comprenant :

des moyens (23') permettant d'extraire un échantillon de signal caractéristique du tracé temporel des impulsions de signaux ou du signal continu reçu dans un intervalle de temps prédéterminé et
des moyens (23") permettant de comparer l'échantillon de signal caractéristique avec un ou plusieurs échantillons de référence caractéristiques, la présence d'air étant déterminée lorsque l'échantillon de signal caractéristique diffère d'au moins un échantillon de référence caractéristique d'un facteur prédéterminé, **caractérisé en ce que** les moyens (23") sont conçus pour la comparaison de l'échantillon de signal caractéristique avec un ou plusieurs échantillons de référence caractéristiques de façon à ce que, à partir de l'échantillon de signal caractéristique, une ou plusieurs grandeurs statistiques caractéristiques sont déterminées et sont comparées avec une ou plusieurs grandeurs statistiques de référence.

**9.** Dispositif selon la revendication 8, **caractérisé en ce que** les moyens (23') sont conçus pour l'extraction d'un échantillon de signal de façon à ce que les échantillons de signaux caractéristiques sont déterminés en continu dans une série d'intervalles de temps et comparés chacun avec un ou plusieurs échantillons de référence.

**10.** Dispositif selon la revendication 8 ou 9, **caractérisé en ce que** les moyens (23") sont conçus pour la comparaison de l'échantillon de signal caractéristique avec un ou plusieurs échantillons de référence caractéristiques de façon à ce que la grandeur statistique caractéristique calculée soit la variance.

**11.** Dispositif selon l'une des revendications 8 à 10, **caractérisé en ce que** les moyens (23 ") sont conçus pour la comparaison de l'échantillon de signal caractéristique avec un ou plusieurs échantillons de référence caractéristiques de façon à ce que les échantillons de signaux caractéristiques sont déterminés en continu dans une série d'intervalles de temps et comparés chacun avec un ou plusieurs échantillons de référence, la valeur moyenne calculée dans un ou plusieurs intervalles de temps précédents est utilisée pour calculer la variance en tant que grandeur statistique caractéristique.

**12.** Dispositif selon l'une des revendications 8 à 11, **caractérisé en ce que** les moyens (23 ") sont conçus pour extraire un échantillon de signal de façon à ce que, en tant qu'échantillon de signal caractéristique, la série, apparaissant dans un intervalle de temps prédéterminé, des amplitudes maximales des impulsions de signaux est extraite.

**13.** Dispositif selon l'une des revendications 8 à 12, **caractérisé en ce que** les moyens d'envoi de la série d'impulsions de signaux ou du signal continu sont un émetteur d'ultrasons (19) et les moyens de réception des impulsions ultrasonores sont un récepteur d'ultrasons (20).

**14.** Dispositif du traitement extracorporel du sang avec une circulation sanguine extracorporelle et un dispositif selon l'une des revendications 8 à 13, la présence d'air dans la circulation sanguine extracorporelle étant surveillée.

**Fig. 1**

**Fig. 2**

EP 1 886 130 B1

## Fig. 3

## Fig. 4a

Zoom (50ms)

## Fig. 4b

## Fig. 5a

Zoom (50ms)

## Fig. 5b

**IN DER BESCHREIBUNG AUFGEFÜHRTE DOKUMENTE**

**In der Beschreibung aufgeführte Patentdokumente**

- EP 1182452 A2

- US 5824881 A

**In der Beschreibung aufgeführte Nicht-Patentliteratur**

- **DROSTE DW ; KÜHNE K ; SCHAEFER RM ; RINGELSTEIN EB.** Detection of microemboli in the subclavian vein of patients undergoing haemodialysis an haemodiafiltration using pulsed Doppler ultrasound. *Nephrol Dial Transplant,* 2002, vol. 17, 462-466